# EUROPEAN PATENT APPLICATION

(11) **EP 4 710 852 A1**
(43) Date of publication of application: **18.03.2026**
(21) Application number: 24382991.8
(22) Date of filing: 17.09.2024
(51) Int. Cl.: A61B 5/11, A61B 5/16, A61B 5/00, A63F 13/00, G16H 50/30

(54) **METHODS, DEVICE AND STORAGE MEDIUM FOR THE EVALUATION OF COGNITIVE ABILITIES**

(71) Applicant: Indivi AG, 4051 Basel (CH)
(72) Inventor: BARTHOLOMÉ, Emmanuel Jacques, 6330 Cham (CH); BERNASCONI, Corrado Angelo, 5000 Aarau (CH); REYES PUPO, Óscar Gabriel, 14011 Córdoba (ES); LUQUE CÓRDOBA, Carlos, 14005 Córdoba (ES); RODRÍGUEZ ROMERO, Antonio Jesús, 14004 Córdoba (ES); BELACHEW, Shibeshih Mitiku, 8044 Zürich (CH); DUPONT, Ghilhem Bruno André, 4051 Basel (CH)
(74) Representative: Vossius & Partner Patentanwälte Rechtsanwälte mbB

(57) **Abstract**

In an aspect, the disclosure relates to a method for determining a user's cognitive abilities. The method comprises providing, by a mobile device, a sequence of first tasks, to be performed by a user of the mobile device, based on displaying one or more first objects at respective one or more first positions on a touchscreen display of the mobile device, and detecting, while the user is performing the sequence of first tasks, one or more user inputs related to the one or more first objects on the touchscreen display. The method further comprises determining, for each respective first task of the sequence of first tasks, a user performance characteristic based on spatial and temporal properties of the one or more user inputs on the touchscreen display, and evaluating, for each respective first task of the sequence of first tasks, a cognitive ability of the user based on a comparison of the determined performance characteristic of the respective first task with the determined performance characteristic of a preceding first task or the determined performance characteristics of a plurality of preceding first tasks of the sequence of the first tasks.

## Description

### Field of the disclosure

The present disclosure relates to methods for determining cognitive abilities of a user of a mobile device. The disclosure also relates to a corresponding computer program product, computer-readable storage medium, and mobile device.

In particular, the disclosure relates to a method for determining user cognitive abilities based on a user-specific and adjustable baseline susceptible to sensitize the detection, diagnosis, measurement or monitoring of cognitive impairment at early stages of neurodegenerative diseases.

### Technical background

As the average life expectancy of the human population increases, the prevalence of neurogenerative diseases affecting human cognitive abilities such as, for example, Alzheimer's disease, Parkinson's disease or multiple sclerosis has increased. Consequently, improved methods for evaluating human cognitive abilities have gained relevance. However, methods of the current state of the art exhibit several drawbacks.

As a general standard, a person's cognitive abilities are evaluated in a clinical environment where one or more cognitive tests are administered in a healthcare facility, typically under supervision of medical personnel, which introduces an observation bias. These in-clinic supervised tests are also subjective to a large extent and vary between successive evaluations, rendering an evaluation of a person's test performance imprecise. Additionally, requiring tests to be administered in-clinic or at a point-of-care is cumbersome and time consuming, leading to infrequent visits and tests. As a result, test data is limited, thus further rendering an evaluation of a person's test performance imprecise.

Furthermore, current approaches employ neuropsychological tests that are one-dimensional and neither adaptive nor adapted specific to a person. This leads to a one size fits all approach where all individuals are evaluated by the same test configuration at a fixed difficulty level of the task, which does not account for the wide spectrum between subject variability in baseline cognitive abilities of a specific person. In yet another aspect of conventional tests, generally only one or few aspects of a person's cognitive abilities are tested.

With the increased use of personal mobile devices, new methods for evaluating a person's cognitive abilities can allow tests to be frequently and regularly administered in within the comforts of a person's home, which represents a more ecological environment. Such new methods are needed to address the drawbacks of the methods currently employed according to the state of the art, in particular, for improving the sensitivity to detect or diagnose cognitive impairment as seen in neurodegenerative diseases at their earliest stages, i.e., prior to the first subjective complaints and/or reportable symptoms.

### Summary of the disclosure

Some or all of the above problems are solved or at least minimized by the present disclosure. The disclosure is set out by the independent claims. Preferred embodiments are defined by the sub-features of the dependent claims.

In an aspect, the disclosure relates to a method for determining a user's cognitive abilities. The method comprises providing, by a mobile device, a sequence of first tasks, to be performed by a user of the mobile device, based on displaying one or more first objects at respective one or more first positions on a touchscreen display of the mobile device, and detecting, while the user is performing the sequence of first tasks, one or more user inputs related to the one or more first objects on the touchscreen display. The method further comprises determining, for each respective first task of the sequence of first tasks, a user performance characteristic based on spatial and temporal properties of the one or more user inputs on the touchscreen display, and evaluating, for each respective first task of the sequence of first tasks, a cognitive ability of the user based on a comparison of the determined performance characteristic of the respective first task with the determined performance characteristic of a preceding first task or the determined performance characteristics of a plurality of preceding first tasks of the sequence of the first tasks.

When performing tasks, performance characteristics for different users may yield different results due to inherent differences in the cognitive abilities of different users that are unrelated to and hence not indicative of neurodegeneration. Additionally, changes in performance characteristics can be based on natural learning progression when repeatedly performing a task, and hence, these type changes can also not necessarily be considered indicative of neurodegeneration. By comparing a determined performance characteristic with the determined performance characteristic of one or more preceding tasks, the method according to the present disclosure is capable of accounting for individual baseline abilities and natural learning progression of the user, thus improving the evaluation of the cognitive abilities of the user.

Various embodiments may preferably implement the following features:
In embodiments, for a succeeding first task, the level of difficulty of the task is increased or decreased based on whether the performance characteristic(s) for a preceding first task or for a plurality of preceding first tasks is above or below a first threshold value.

Adjusting the difficulty based on a threshold allows the method to account for inherent cognitive abilities of the user, wherein different users may have different inherent cognitive abilities to start with.).

In embodiments, for a succeeding first task, the level of difficulty the task is increased or decreased, preferably only, based on whether a difference between the performance characteristics of two or more preceding first tasks is above or trespass a second threshold value.

Adjusting the difficulty based on a comparison between the performance characteristics of preceding tasks allows the method to account for a natural learning progression of the user, wherein different users may exhibit different learning progression which may or not be indicative of neurodegeneration.

In embodiments, detecting the one or more user inputs on the touchscreen display comprises one or more of detecting a number of touchscreen taps, detecting a time, duration and/or position of one or more taps related to the one or more first objects, detecting one or more tap sequences, detecting a number of swiping gestures, detecting, for each position in a movement path, one or more of a start time, a start position, an end time, an end position, and/or a movement distance of one or more swiping gestures or tap sequences related to the one or more first objects.

This allows quantitative analysis of a user's inputs for quantifying a user's performance via the performance characteristic.

In embodiments, determining a user performance characteristic comprises one or more of determining whether a position of the one or more taps corresponds to the one or more first positions of the one or more first objects, determining a time difference between a time at which the one or more first objects are displayed and a time at which the one or more first taps are detected, determining a time difference between the time at which one or more first taps of a tap sequence are detected, determining whether a movement path of one or more swiping gestures and/or one or more tap sequences corresponds to a predefined movement path, determining the quantified distance between a movement path of one or more swiping gestures and/or one or more tap sequences and a predefined movement path, and/or determining the time, frequency, and/or position information of a movement path of one or more swiping gestures and/or one or more tap sequences.

This allows quantitative analysis of a user's inputs for quantifying a user's performance via the performance characteristic.

In embodiments, providing the first task comprises displaying, on the touchscreen display, one or more position indicators and/or movement path indicators at one or more second positions associated with one or more first objects.

This allows the provision of more complex tasks for determining more diverse aspects of a user's cognitive abilities.

In embodiments, determining a user performance characteristic comprises one or more of determining whether a position of one or more taps correspond to the one or more second positions of the one or more position indicators, determining a time difference between one or more taps corresponding to the one or more second positions of the one or more position indicators and/or movement path indicators and the one or more taps corresponding to the one or more first positions of the one or more first objects, determining whether the one or more swiping gestures correspond to the one or more movement path indicators, and/or determining a total time of one or more swiping gestures that correspond to the one or more movement path indicators.

This allows further detailed quantitative analysis of a user's performance when performing the sequence of tasks for evaluating a user's cognitive abilities.

In embodiments, a first time delay is provided between a time at which the one or more first objects, the one or more position indicators, and/or the one or more path indicators are displayed and a time at which the one or more first taps can be first detected. Additionally, or alternatively, a second time delay is provided between a time at which the one or more first objects, the one or more position indicators, and/or the one or more path indicators are ceased to be displayed and a time at which the one or more first taps can be first detected.

Providing the first and/or the second time delay provides a further challenge to a user when performing the sequence of tasks, as specifies a time a user is challenged to hold instructions or presented solutions to a task to be performed in memory, therefore providing a further quantitative aspect for determining a user's performance characteristic.

In embodiments, a difficulty level of a first task of the sequence of tasks is increased or decreased by one or more of the following: increasing or decreasing a number of the one or more first objects, changing an orientation, shape, and/or size of the one or more first objects, increasing or decreasing a number of position indicators, changing an orientation, shape, and/or size of the one or more position indicators, changing the length and/or orientation of the predefined path, changing the length and/or orientation of the one or more movement path indicators, decreasing or increasing the first time delay, and/or increasing or decreasing the second time delay. Notably, the quantitative parameters which are adjusted to increase or decrease the level of difficulty of a given first task do not modify the type(s) of cognitive function(s) involved in the first task.

This provides quantitative adjustments to adjust the difficulty of a task to be performed by the user.

In embodiments, the method further comprises determining facial features of the user while the user is performing the sequence of first tasks, wherein determining the user performance characteristic for each respective first task of the sequence of first tasks is further based on the determined facial features of the user.

In embodiments, determining facial features of the user comprises tracking eye movement or gaze direction of the user using a camera of the mobile device.

Tracking facial features, eye movement or gaze direction in particular, provides quantitative data for determining a user's performance characteristic.

In embodiments, for each first task of the sequence of first tasks, determining a performance characteristic and/or evaluating, a cognitive ability of the user is performed using a processing algorithm and/or a machine learning, ML, model.

In embodiments, the ML model is further trained using a first data set that is obtained from a plurality of first tasks that have been performed under supervised conditions.

Using a ML model allows improved processing of data that has increased randomness and noise due to a lack of supervision when a user is performing the sequence of tasks, thereby improving the evaluation of the cognitive ability of the user

In embodiments, supervised conditions comprise conditions where the plurality of first tasks are performed under controlled adherence to instructions.

In embodiments, the sequence of first tasks provided by the mobile device is provided to the user in an unsupervised environment.

Training the ML model on data collected in a controlled and/or supervised environment improves the ML model's ability to process random and noisy data collected from user's in under unsupervised conditions.

In embodiments, the method further comprises providing, by the mobile device, a sequence of second tasks, to be performed by the user of the mobile device, based on displaying one or more second objects at respective one or more third positions on the touchscreen display of the mobile device, detecting, while the user is performing the sequence of second tasks, one or more user inputs related to the one or more second objects on the touchscreen display, determining, for each respective second task of the sequence of second tasks, a user performance characteristic based on spatial and temporal properties of the one or more user inputs on touchscreen display, and evaluating, for each respective second task of the sequence of second tasks, a cognitive ability of the user based on a comparison of the determined performance characteristic of the respective second task with the determined performance characteristic of a preceding second task or the determined performance characteristics of a plurality of preceding second tasks of the sequence of the second tasks.

Providing a sequence of second tasks allows to evaluate different aspects of a user's cognitive abilities.

In embodiments, the method further comprises detecting motion of the mobile device within the one or more first task(s) and associated with the one or more user inputs related to the one or more first objects and/or the one or more second objects on the touchscreen display, wherein determining the user performance characteristic is further based on the detected motion of the mobile device.

In another aspect, the disclosure relates to an apparatus comprising a touchscreen display, and a processor configured to perform a method according to any one of above-described embodiments.

In embodiments, the apparatus further comprises one or more inertial sensors, wherein the processor is configured to further detect motion of the mobile device within the one or more first task(s) and associated with the one or more user inputs related to the one or more first objects and/or the one or more second objects on the touchscreen display, wherein determining the user performance characteristic is further based on the detected motion of the mobile device.

Detecting motion of the mobile device while performing cognitively challenging tasks provides further quantitative cognitive-motor interference data for determining a user's performance characteristic, as the user can interact with the mobile device not only via the touchscreen display, but also by voluntarily or involuntarily moving the handheld mobile device.

In yet another aspect, the disclosure relates to detecting, diagnosing or measuring change (decline or improvement) in neurodegenerative diseases affecting cognitive abilities potentially prior to the first subjective complaints or reported symptoms, based on the user performance characteristic(s) according to any one of above-described embodiments.

In embodiments, the method according to any one of the preceding embodiments can be used to determine a user performance characteristic(s) in a first task or a plurality of first task(s) to detect, diagnose, or measure change (decline or improvement) in a neurodegenerative disease such as, but not exclusively, multiple sclerosis, Alzheimer's disease and related dementias, fronto-temporal dementia, Parkinson's disease, dementia with Lewy bodies, multiple system atrophy, dementias biologically defined by brain accumulation of amyloid-beta, TDP-43 inclusions, alpha-synuclein or other proteinopathies.

In yet another aspect, the disclosure relates to a computer program product comprising instructions that, when executed by a processor of an apparatus as described in embodiments above, cause the processor to perform a method according to any one of the above-described embodiments.

In yet another aspect, the disclosure relates to a readable storage medium comprising a computer program product comprising instructions that, when executed by a processor of an apparatus as described in embodiments above, cause the processor to perform a method according to any one of the above-described embodiments.

Other aspects, features, and advantages will be apparent from the summary above, as well as from the description that follows, including the figures and the claims.

Embodiments of the present disclosure will now be described by way of example only and with reference to the following accompanying drawings. In the figures, the same reference numerals denote the same or similar elements.

### Brief description of the drawings

Fig. 1 shows a schematic illustration of tasks provided to a user according to embodiments of the present disclosure.
Fig. 2 shows a schematic illustration of tasks provided to a user according to further embodiments of the present disclosure.
Figs. 3A and 3B show a schematic illustration of tasks provided to a user according to yet further embodiments of the present disclosure.

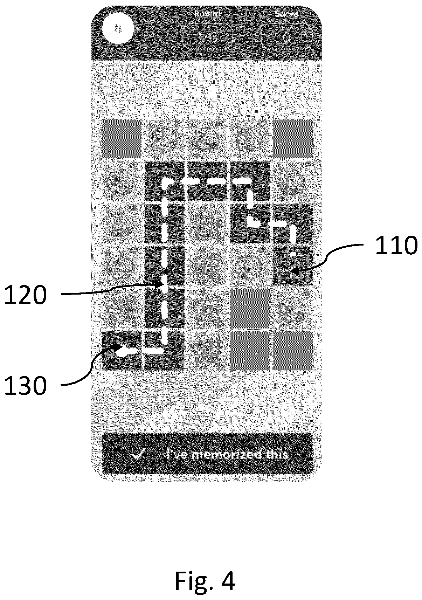

Fig. 4 shows a schematic illustration of tasks provided to a user according to yet further embodiments of the present disclosure.
Figs. 5A and 5B show a schematic illustration of methods according to embodiments of the present disclosure.

### Detailed description

In embodiments of the present disclosure, one or more sequences of tasks are provided to a user via a mobile electronic device. A mobile electronic device may be any electric device that can be carried and/or moved by a user without assistance of machinery or other technical means. A mobile device may be a mobile computing device such as smartphone, smartwatch, tablet, or laptop computer. The mobile device comprises a touchscreen display, one or more process processors, and computer-readable memory. The mobile device may further comprise communication means, preferably wireless communication means, for communication with one or more of remote device(s), servers, and/or (mobile) communication network(s).

In embodiments, the mobile device comprises sensors to detect motion of the mobile device. The sensor may comprise inertial sensors such as, for example, accelerometers, gyroscopes, magnetometers. The mobile device may further be provided with global positioning system, GPS, capability. In embodiments, the mobile device may also further comprise optical detection means such as a camera, preferably a front-facing (depth-sensing) camera, for determining facial features such as facial expressions, eye movement or gaze direction of the user of the mobile device.

Initially, prior to a user performing a task, instructions on how to perform the task may be provided to the user. For example, text instructions may be displayed on the touchscreen display, or audio instructions may be provided to the user.

Generally, different tasks provided to the user may be configured for evaluating different aspects of the user's cognitive abilities. Here, cognitive abilities of the user can be broadly categorized into different domains that comprise, but are not limited to, information processing speed, short-term memory (e.g., visuo-spatial), executive functioning, working memory, procedural memory, mental flexibility, divided attention, and/or sustained attention of the user.

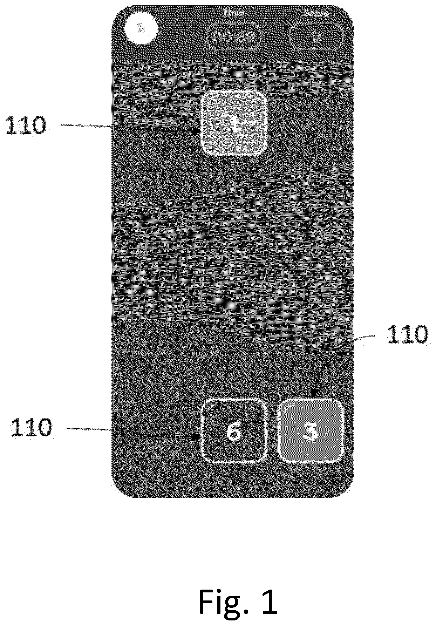

**Fig. 1** shows a schematic illustration of a first example task provided to a user on the touchscreen display of a mobile device, according to embodiments of the present disclosure. Here, the task provided to the user may be configured to allow evaluation of the information processing speed and/or the ability for sustained attention of the user.

A plurality of first objects 110 are displayed on the touchscreen display, wherein each of the plurality of first objects 110 indicates information that can be sequentially or chronologically ordered. For example, first objects 110 may indicate numbers or letters that can be sequentially ordered in an ascending or a descending order. For numbers, this order can mean in the order of increasing numbers or in the order of decreasing numbers. For letters, this order can mean following the alphabetical order or going against the alphabetical order. For performing the task, the user is required to select first objects 110 in a respective order that can be given via the instructions prior to performing the task. The user preferably selects a first object 110 by tapping one or more fingers onto a position on the touchscreen display at which the first object 110 is displayed on the touchscreen display.

While the user is performing the task, for each finger tap, input data comprising one or more of a time, duration, and/or position of the tap may be collected and recorded in a data storage.

Based on the collected input data, a performance characteristic of the user for the performed task may be determined. Notably, it is to be determined whether the user has performed the task according to provided instructions, and how well the user was able to perform the task. For this one or more of the following may be determined based on the collected input data.

For example, it may be determined whether the user was able to correctly select respective first objects 110 in the appropriate order provided in the instructions by determining whether positions of finger taps correspond to the positions at which respective first objects 110 were displayed on the touchscreen display. Additionally, or alternatively, the total time from a first tap corresponding to a first object 110 to be first selected according to a given order to a last tap corresponding to a first object 110 to be last selected according to the given order may be determined to determine how quickly the user was able to perform the task. Additionally, or alternatively, the average time interval between successive correct taps corresponding to multiple first objects 110 to be selected according to a given order may be determined to determine how accurately and quickly the user was able to perform the task. Additionally, or alternatively, the total number of successive correct taps corresponding to multiple successive objects 110 to be selected according to a given order may be determined to determine how accurately and quickly the user was able to perform the task. Additionally, or alternatively, a time interval from a time when instructions on how to perform the task are ceased to be displayed to a time of a first tap corresponding to the first object 110 to be first selected may be determined to determine how quickly the user was able to process the information provided by the instructions to start performing the task, which is indicative of the user's (psycho-motor) reaction time.

Based on these determinations, a performance characteristic can be determined. The performance characteristic can be given in any suitable format. A determined correct number of taps may be compared against a total number of first objects and assigned to a score based on the relative and/or total number of correct taps. Determined time intervals, time intervals between successive correct taps in particular, may be assigned to a score, with longer time intervals assigned to lower score value and shorter time intervals assigned to higher score. As an example, the score can be given as any suitable combination of alphanumerical symbols. The performance characteristic of the task can be a (weighted) sum or (weighted) average of different scores determined for the task.

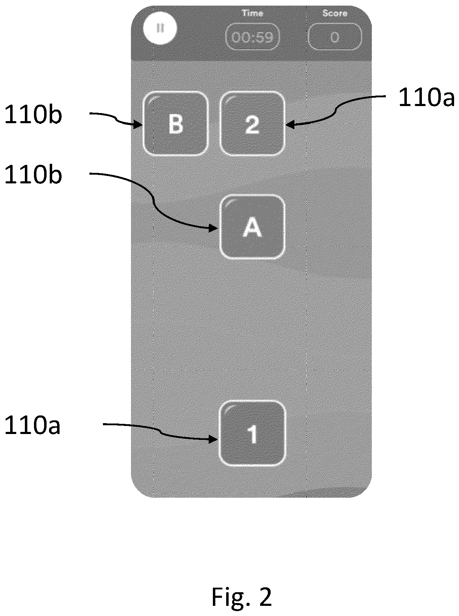

**Fig. 2** shows a schematic illustration of a second example task provided to a user on the touchscreen display of a mobile device, according to embodiments of the present disclosure. The second example task represents a modification of the first example task and may be configured to test a user's ability to deal with situations where divided attention and mental flexibility is required.

Here, a first group of a plurality of first objects 110a, and a second group of a plurality of first objects 110b is displayed on the touchscreen display. The objects 110a of the first group can differ from objects 110b of the second group by type, orientation, size, color or any other suitable differentiating feature. For example, while the first group may indicate numbers, the second group may indicate letters. The second example task may require the user to select the objects 110a of the first group in a respectively given order, and additionally select the objects 110b of the second group in a respectively given order. Additionally, the user may be required to select the objects 110a of the first group in the respective order prior to selecting the objects 110b of the second group in the respective order. Alternatively, the user may be required to select objects 110a of the first group and objects 110b of the second group in alternating fashion but maintain the respectively given orders instructed for each group. For example, the user may be required to select numbers [10, 6, 1] in descending order, in alternation with selecting letters [B, G, X] in alphabetical order. The user must then first select the largest displayed number [10], then subsequently select the letter [B] that occurs first in the alphabet, then subsequently the second largest displayed number [6], and then subsequently select the letter [G] that occurs second the in the alphabet and so on.

The collected input data may be the same input data as described above for the first example task. For determining a performance characteristic, the same determination as described for the first example task may be performed. Additionally, or alternatively, a time interval between a tap corresponding to an object 110a of the first group and a subsequent tap corresponding to an object 110b of the second group, and/or vice versa, may be determined to determine the user's ability to deal with divided attention and mental flexibility, wherein shorter time intervals may be assigned to higher scores and longer time intervals assigned to lower scores.

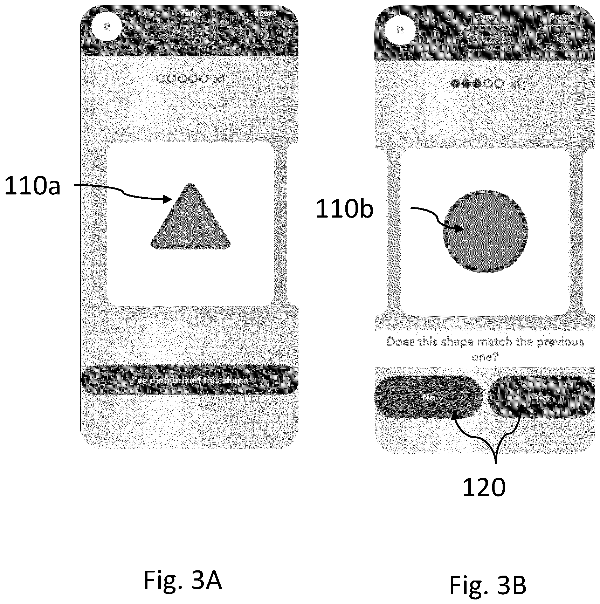

**Figs. 3A and 3B** show a schematic illustration of a third example task provided to a user on the touchscreen display of a mobile device, according to embodiments of the present disclosure. Here, the task provided to the user may be configured to allow evaluation of the working memory of the user.

First, as shown in Fig. 3A, an object 110a of a first group of a plurality of first objects is displayed to the user. After a certain amount of time that may be predefined, as shown in Fig. 3B, one or more objects 110b of a second group of first objects is displayed to the user, and the object 110a of the first grouped is ceased to be displayed. An object 110b of the second group 110b may be displayed sequentially one by one. After an object 110b of the second group is displayed, the user is required to provide an input as to whetherthe displayed second object 110b corresponds to or matches the 1-back previously displayed object 110a of the first group, i.e., the displayed object 110a immediately preceding the displayed second object 110b. Alternatively, after an object 110b of the second group is displayed, the user may be required to provide an input as to whether the displayed second object 110b corresponds to or matches the 2-back (or N-back) previously displayed object of a prior group. An input indicating a positive correspondence/match or a negative correspondence/match can be given by tapping a position indicator 120 displayed at predefined positions on the display (e.g., "yes"/"no" buttons) or by performing a respective swiping gesture (e.g., an upwards swiping gesture for "yes" and a downwards swiping gesture for "no", or vice versa).

Collected input data may be input data as described above in the context of embodiments illustrated in Figs. 1 and 2. Additionally, or alternatively, one or more swipe gestures may be detected as inputs, and a performance characteristic may be determined based on whether a direction of the swiping gestures corresponds to one or more predetermined directions. For determining a performance characteristic, the same determination as described for the first and/or second example tasks may be performed.

**Fig**. **4** shows a schematic illustration of a fourth example task provided to a user on the touchscreen display of a mobile device, according to embodiments of the present disclosure. Here, the task provided to the user may be configured to allow evaluation of the visuo-spatial short-term memory of the user.

A first object 110 indicating a destination position is displayed on the touchscreen display in addition to a movement path indicator 120 indicating a starting position and a movement path along which the destination position is to be reached from the starting position 130 indicated by the movement path indicator 120. The first object 110, the movement path indicator 120 and the starting position 130 may be arranged on a grid of a predefined size. After a time interval, which may be predefined, the movement path indicator 120 and optionally the first object 110 are ceased to be displayed. The user is then required to re-trace the movement path from the starting position 130 to the destination position 110. Re-tracing the movement can be performed by one or more swiping gestures or a sequence of taps that, when correctly performed, follow along the path previously indicated by the movement path indicator. Here, a tap sequence may correspond to a temporal sequence of taps on adjacent positions on the provided grid performed within a certain time interval. In case a user is unable to re-trace the movement path, the user may be prompted, preferably a predefined number of times, to repeat re-tracing of the movement path.

Collected input data may be input data as described above in the context of embodiments illustrated in Figs. 1 to 3B. Additionally, one or more swiping gestures and/or tap sequences may be recorded. Notably, one or more of start time, start position, end time, end position, and/or movement distance of one or more swiping gestures or tap sequences may be detected.

For determining a performance characteristic, the same determinations as described for the first, second, and third example tasks may be performed. Additionally, or alternatively, the following may be determined.

A time interval between a time, at which the first object 110, the movement path indicator 120 and the starting position 130 are first displayed and a time, at which the user provides an input such as, e.g., tapping a predefined button on the touchscreen display to cease display of the movement path indicator, is determined to determine a score indicative of the user's ability to memorize visuo-spatial information. Additionally, or alternatively, a time interval from a time, when display of the movement path indicator 120 is ceased, and an end time of a swiping gesture or tap sequence may be determined to determine a score indicative of the user's ability to quickly perform the given task. Additionally, or alternatively, it may be determined whether a movement path of the one or more swiping gestures or of a tap sequence corresponds to the path previously indicated by the movement path indicator 120. Additionally, or alternatively, it may be determined how much a movement path of the one or more swiping gestures or of a tap sequence deviates from the path previously indicated by the movement path indicator 120. For example, the proportion of correctly connected squares of the grid that belong to the movement path indicator 120 can be quantified. Mutual information analysis between the reconstructed path of the one or more swiping gestures or tap sequence and the reference movement path indicator 120 can also be used to this effect. The number of times the user attempted to re-trace the movement path until the user was able to correctly re-trace the movement path may be determined. The frequency of taps corresponding to a determined tap sequence may further be determined. Shorter time intervals or a lesser number of tries to correctly re-trace the movement path may be assigned to a higher score, while longer time intervals, a larger number of tries to correctly re-trace the movement path, or failure to correctly re-trace the movement path may be assigned to a lower score. As described above, the performance metric maybe a (weighted) sum or (weighted) average or different scores determined for the performed task.

In any one of the above example tasks or combination of example tasks, further information regarding the motion of the mobile device while the task is being performed may be detected and recorded as input data. As an example, an oscillating motion of the mobile device due to, e.g., the mobile device being held by the user during performing the task, or motion of the mobile device during a finger tap and/or swiping gestures may be detected and recorded. A larger oscillating motion of the mobile device while being held or a larger motion of the mobile device during a finger tap and/or swiping gestures can be indicative of difficulties of the user in using motor skills when performing a given cognitive task, which can be indicative of cognitive-motor interference. A smaller motion in both cases may respectively be assigned to a higher score, while a larger motion may be respectively assigned to a lower score.

Additionally, or alternatively, in any one of the above example tasks or combination of example tasks, further information regarding facial features such as facial expressions, eye movement/gaze direction using a (depth-sensing) camera of the mobile device may be detected and recorded as input data. For example, it may be determined whether a user's gaze is directed to a position of a first object 110 or a tap corresponding to a first object 110 during a tap, and/or whether a user's gaze direction changes, oscillates, and/or fluctuates in between taps or swiping gestures, and/or during a time interval, where the user is processing/memorizing information provided by, e.g., instructions, without interacting with the touchscreen display.

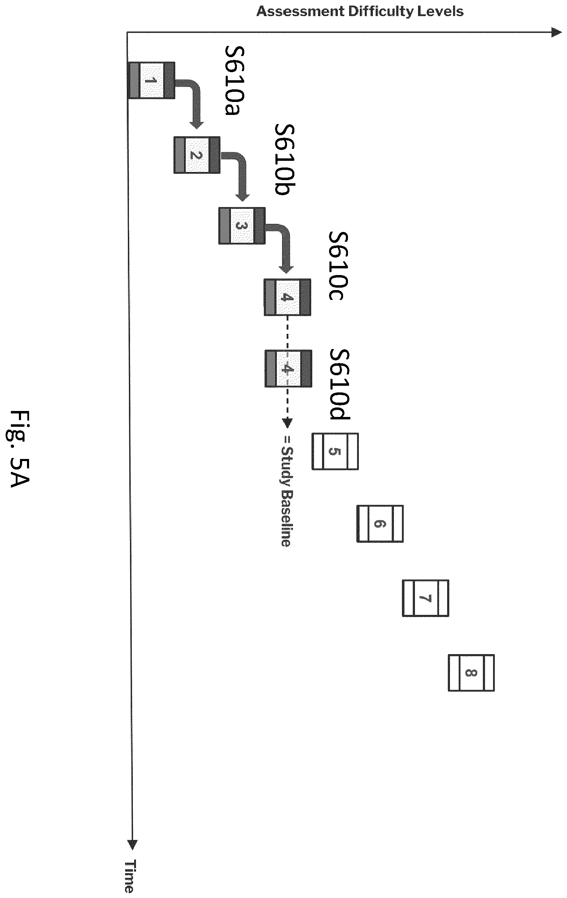

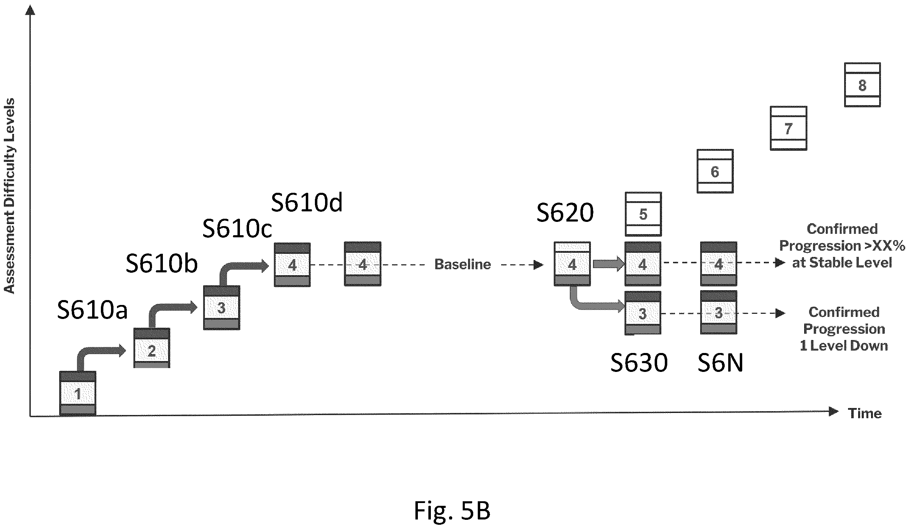

**Figs. 5A** **and** **5B** illustrate a method according to the present disclosure for evaluating a cognitive ability of a user of the mobile device. The method comprises providing one or more tasks to be performed on a touchscreen display of the mobile device to a user.

The one or more respectively provided tasks, determined user inputs, and determined performance characteristics are preferably as described above for any one of the first to fourth example tasks. Notably, the method comprises providing a sequence of first tasks to be performed by the user.

In steps S610a to S610d, a baseline performance characteristic specific to the user is determined. In step S610a, a first task of the sequence is provided to the user. The task is performed by the user, and a performance characteristic is determined as described above. In step S610a, a succeeding first task of the sequence is provided to the user. The succeeding task may be provided to the user with a time delay relative to the preceding task, wherein the time delay may preferably be hours or days.

Depending on the performance characteristic of the preceding first task of step S610a, the difficulty level of the first task provided to the user in step S610b may be increased from an initial level, e.g., level 1, to a higher level, e.g., level 2. For example, if the performance characteristic determined for the preceding task is above a first threshold that may be predefined, the first task of step S610a can be considered too simple or not challenging enough for the baseline cognitive capacity of the user.

After step S610b, a further succeeding first task is provided to the user in step S610c, wherein step S610c may be performed after a further time delay as described for preceding step S610b. The difficulty level of the first task provided in step S610c may be increased from level 2 to a higher level, e.g., level 3, based on the performance characteristic of the preceding task performed in preceding step S610b or based on the performance characteristics of multiple preceding tasks performed in multiple preceding steps. If the performance characteristic of the task of preceding step S610b remains above the first threshold (or an alternative second threshold), this can mean that the increase in difficulty in step S610b was insufficient, and the difficulty level for the first task in step S610c is further increased. For example, if the performance characteristic or the average of the performance characteristics of two or more preceding steps, here merely exemplary of steps S610b and S610a, is in, e.g., the 75% percentile of overall performance characteristics determined for, e.g., a plurality of different users, the difficulty level is increased.

Additionally, or alternatively, the change in performance characteristic for first tasks of preceding steps can be compared for determining whether or not to adjust the difficulty level for the first task of step S610c. If the performance characteristic of step S610b is higher than the performance characteristic S610a, and/or a change in performance characteristic of step S610b compared to step S610a is above a second threshold, this can be indicative of a natural learning progression of the user in performing the first task. In such case, the difficulty level for the first task provided in step S610c can be further increased.

In some embodiments, the user remains at the same level of difficulty during two or more successive first tasks before the user performance characteristic may attain a first or second threshold indicative of a learning progression, which can trigger a subsequent increase in difficulty level when providing a succeeding first task. The provision of the first task and adjustment of the difficulty level can be repeated in successive steps as described in steps S610a to S610c until the number of steps reaches a predefined maximum number of tasks that may be provided per sequence tasks. Additionally, or alternatively, the provision of the first task and adjustment of the difficulty level can be repeated until, in a step S610d, the performance characteristic for step S610d is below the first threshold or the change in the performance characteristic of a preceding step S610c and the performance characteristic of step S610d is below the second threshold. As an example, the second threshold can be determined by an expected range of variability at a specific confidence interval for this given difficulty level based on a reference population of stable users. In this case, the performance characteristic determined for step S610d using a first task at the respective difficulty level, e.g., level 4, establishes the baseline performance characteristic for evaluating the cognitive abilities of the user. In some embodiments, the transition between difficulty levels is possible only based on averaging user performance characteristics on two or more first tasks executed at the same difficulty level.

Determining a baseline performance characteristic by adjusting the difficulty level also comprises decreasing the difficulty level if the performance characteristic for one or more preceding steps is below a third threshold or if a decrease in the performance characteristic between the performance characteristics of two or more preceding steps is above a fourth threshold. For example, if the performance characteristic or the average of the performance characteristics of two or more preceding steps, here merely exemplary of steps S610b and S610, is in, e.g., the 25% percentile of overall performance characteristics determined for, e.g., a plurality of different users, the difficulty level is decreased.

As illustrated in Fig. 5B, once the baseline performance characteristic is established, further provision of the first task to the user in succeeding steps S630 to S6N may be used to determine a change in the cognitive abilities of the user, wherein changes in the performance characteristic may be indicative of a change in the cognitive abilities of the user.

In succeeding steps S630 to S6N, the difficulty level of the provided first task preferably remains unchanged. However, in cases where a significant decrease in performance characteristics and hence a significant degradation of the cognitive abilities of the user is determined, the difficulty level for succeeding first tasks provided to the user may be decreased. This can "reset" the baseline performance characteristic and increase the resolution, with which further changes in the performance characteristic can be determined.

Adjusting the difficulty level can be performed in several ways. For example, in the first and second example embodiments, the number of first objects 110 can be increased or decreased to increase or decrease the difficulty level. In the third example embodiment, the number of different shapes and colors of the first objects 110 can be increased or decreased to increase or decrease the difficulty level. In the fourth example embodiment, the number of position indicators or movement path indicators 120 may be increased or decreased to increase or decrease the difficulty level. Additionally, or alternatively, the orientation, shape, and/or size of the one or more position indicators 120, the length and/or orientation of the predefined path from the starting position to the destination position, and/or the length and/or orientation of the one or more movement path indicators 120 may be changed to adjust the difficulty level. Additionally, or alternatively, the size of the grid on which the first objects 110 and the position or movement path indicators 120 are displayed may be increased or decreased to increase or decrease the difficulty level. In any one of the example embodiments, the orientation, shape, and/or size of the displayed first objects 110, 110a, 110b may be changed. For example, objects may be blurred, rotated, enlarged or made smaller, or otherwise altered.

Additionally, or alternatively, a predefined time delay between a time, when instructions or movement path indicators 120 indicating a correct movement path are first displayed, and a time, when the instructions or movement path indicators 120 are ceased to be displayed, can be introduced. Increasing or decreasing this time delay increases or decreases the time for memorization and/or processing of information regarding the task to be performed. Hence, a decrease or increase in this time delay can be used to increase or decrease the difficulty level of the task to be performed.

Additionally, or alternatively, a predefined time delay between a time, when instructions or movement path indicators 120 indicating a correct movement path are ceased to be displayed, and a time, when the user can begin performing the task by providing inputs to the mobile device, can be introduced. Increasing or decreasing this time delay increases or decreases the time the user is forced to memorize provided information regarding the task to be performed. Hence, an increase or decrease in this time delay can be used to increase or decrease the difficulty level of the task to be performed.

The sequence of first tasks may be provided to a user in a sequence of task sessions. A task session comprises a first task of the sequence of first tasks, and comprises further tasks for further sequences of tasks, wherein the further tasks are preferably different from the first task. For example, a task session may comprise providing the first, and/or second, and/or third, and/or fourth example tasks or any combination thereof to the user. While each task session of the sequence of task sessions comprises the same combination of tasks, the order in which the tasks are provided within a task session may be different for each task session. However, while a task session comprises a plurality of different tasks belonging to respective different sequences of tasks, a cognitive ability of the user is preferably evaluated for each respective sequence of task independently.

In embodiments, determining a performance characteristic and/or evaluating a cognitive ability of the user is performed using a processing algorithm and/or a machine learning, model. In case of a machine learning model, the model can be trained using a first data set that is obtained from a plurality of first tasks that have been performed under supervised conditions. This allows the machine learning model to better process input data that has increased randomness and noise due to a lack of supervision when the user is performing the sequence of tasks using the mobile device.

While various embodiments of the present disclosure have been described above, it should be understood that they have been presented by way of example only, and not by way of limitation. Likewise, the various diagrams may depict an example architectural or configuration, which are provided to enable persons of ordinary skill in the art to understand exemplary features and functions of the present disclosure. Such persons would understand, however, that the present disclosure is not restricted to the illustrated example architectures or configurations but can be implemented using a variety of alternative architectures and configurations. Additionally, as would be understood by persons of ordinary skill in the art, one or more features of one embodiment can be combined with one or more features of another embodiment described herein. Thus, the breadth and scope of the present disclosure should not be limited by any one of the above-described exemplary embodiments.

It is also understood that any reference to an element herein using a designation such as "first," "second," and so forth does not generally limit the quantity or order of those elements. Rather, these designations can be used herein as a convenient means of distinguishing between two or more elements or instances of an element. Thus, a reference to first and second elements does not mean that only two elements can be employed, or that the first element must precede the second element in some manner.

A skilled person would further appreciate that any one of the various illustrative logical blocks, units, processors, means, circuits, methods and functions described in connection with the aspects disclosed herein can be implemented by electronic hardware (e.g., a digital implementation, an analog implementation, or a combination of the two), firmware, various forms of program or design code incorporating instructions (which can be referred to herein, for convenience, as "software" or a "software unit"), or any combination of these techniques.

To clearly illustrate this interchangeability of hardware, firmware and software, various illustrative components and steps have been described above generally in terms of their functionality. Whether such functionality is implemented as hardware, firmware or software, or a combination of these techniques, depends upon the particular application and design constraints imposed on the overall system. Skilled artisans can implement the described functionality in various ways for each particular application, but such implementation decisions do not cause a departure from the scope of the present disclosure. In accordance with various embodiments, a processor, device, component, circuit, structure, machine, unit, etc. can be configured to perform one or more of the functions described herein. The term "configured to" or "configured for" as used herein with respect to a specified operation or function refers to a processor, device, component, circuit, structure, machine, unit, etc. that is physically constructed, programmed and/or arranged to perform the specified operation or function.

A processor can be a microprocessor, but in the alternative, the processor can be any conventional processor, controller, or state machine. A processor can also be implemented as a combination of computing devices, or any other suitable configuration to perform the functions described herein. If implemented in software, the functions can be stored as one or more instructions or code on a computer-readable medium. Thus, the steps of a method or algorithm disclosed herein can be implemented as software stored on a computer-readable medium.

Computer-readable media includes both computer storage media and communication media including any medium that can be enabled to transfer a computer program or code from one place to another. A storage media can be any available media that can be accessed by a computer. By way of example, and not limitation, such computer-readable media can include RAM, ROM, EEPROM, CD-ROM or other optical disk storage, magnetic disk storage or other magnetic storage devices, or any other medium that can be used to store desired program code in the form of instructions or data structures and that can be accessed by a computer.

Additionally, memory or other storage, as well as communication components, may be employed in embodiments of the present disclosure. It will be appreciated that, for clarity purposes, the above description has described embodiments of the present disclosure with reference to different functional units and processors. However, it will be apparent that any suitable distribution of functionality between different functional units, processing logic elements or domains may be used without detracting from the present disclosure.

Various modifications to the implementations described in this disclosure will be readily apparent to those skilled in the art, and the general principles defined herein can be applied to other implementations without departing from the scope of this disclosure. Thus, the disclosure is not intended to be limited to the implementations shown herein but is to be accorded the widest scope consistent with the novel features and principles disclosed herein, as recited in the claims below.

## Claims

1. A method for determining a user's cognitive abilities, the method comprising:
providing, by a mobile device, a sequence of first tasks, to be performed by a user of the mobile device, based on displaying one or more first objects at respective one or more first positions on a touchscreen display of the mobile device;
detecting, while the user is performing the sequence of first tasks, one or more user inputs related to the one or more first objects on the touchscreen display;
determining, for each respective first task of the sequence of first tasks, a user performance characteristic based on spatial and temporal properties of the one or more user inputs on the touchscreen display; and
evaluating, for each respective first task of the sequence of first tasks, a cognitive ability of the user based on a comparison of the determined performance characteristic of the respective first task with the determined performance characteristic of a preceding first task or the determined performance characteristics of a plurality of preceding first tasks of the sequence of the first tasks.

2. The method according to the preceding claim, wherein for a succeeding first task, the level of difficulty of the task is increased or decreased based on whether the performance characteristic(s) for a preceding first task or for a plurality of preceding first tasks is above or below a first threshold value.

3. The method according to any one of the preceding claims, wherein for a succeeding first task, the level of difficulty the task is increased or decreased based on whether a difference between the performance characteristics of two or more preceding first tasks is above or trespasses a second threshold value.

4. The method according to any one of the preceding claims, wherein detecting the one or more user inputs on the touchscreen display comprises one or more of:
detecting a number of touchscreen taps;
detecting a time, duration, and/or position of one or more taps related to the one or more first objects;
detecting one or more tap sequences;
detecting a number of swiping gestures;
detecting, for each position in a movement path, one or more of a start time, a start position, an end time, an end position, and/or a movement distance of one or more swiping gestures or tap sequences related to the one or more first objects,

5. The method according to any one of the preceding claims, wherein determining a user performance characteristic comprises one or more of:
determining whether a position of the one or more taps corresponds to the one or more first positions of the one or more first objects;
determining a time difference between a time at which the one or more first objects are displayed and a time at which the one or more first taps are detected;
determining a time difference between the time at which one or more first taps of a tap sequence are detected;
determining whether a movement path of one or more swiping gestures and/or one or more tap sequences corresponds to a predefined movement path;
determining the quantified distance between a movement path of one or more swiping gestures and/or one or more tap sequences and/or a predefined movement path;
determining the time, frequency, and/or position information of a movement path of one or more swiping gestures and/or one or more tap sequences.

6. The method according to any one of the preceding claims, wherein providing the first task comprises displaying, on the touchscreen display, one or more position indicators and/or movement path indicators at one or more second positions associated with one or more first objects.

7. The method according to the preceding claim, wherein determining a user performance characteristic comprises one or more of:
determining whether a position of one or more taps correspond to the one or more second positions of the one or more position indicators;
determining a time difference between one or more taps corresponding to the one or more second positions of the one or more position indicators and/or movement path indicators and the one or more taps corresponding to the one or more first positions of the one or more first objects;
determining whether the one or more swiping gestures correspond to the one or more movement path indicators;
determine a total time of one or more swiping gestures that correspond to the one or more movement path indicators.

8. The method according to any one of the preceding claims, wherein a first time delay is provided between a time at which the one or more first objects, the one or more position indicators, and/or the one or more path indicators are displayed and a time at which the one or more first taps can be first detected; and/or
wherein a second time delay is provided between a time at which the one or more first objects, the one or more position indicators, and/or the one or more path indicators are ceased to be displayed and a time at which the one or more first taps can be first detected.

9. The method according to any one of claims 2 to 8, when dependent on claim 2, wherein a difficulty level of a first task of the sequence of tasks is increased or decreased by one or more of the following:
increasing or decreasing a number of the one or more first objects;
changing an orientation, shape, and/or size of the one or more first objects;
increasing or decreasing a number of position indicators;
changing an orientation, shape, and/or size of the one or more position indicators;
changing the length and/or orientation of the predefined path;
changing the length and/or orientation of the one or more movement path indicators;
decreasing or increasing the first time delay;
increasing or decreasing the second time delay.

10. The method according to the preceding claim, wherein supervised conditions comprise conditions where the plurality of first tasks are performed under controlled adherence to instructions.

11. The method according to any one of the preceding claims, wherein the sequence of first tasks provided by the mobile device is provided to the user in an unsupervised environment.

12. The method according to any one of the preceding claims, further comprising:
providing, by the mobile device, a sequence of second tasks, to be performed by the user of the mobile device, based on displaying one or more second objects at respective one or more third positions on the touchscreen display of the mobile device;
detecting, while the user is performing the sequence of second tasks, one or more user inputs related to the one or more second objects on the touchscreen display;
determining, for each respective second task of the sequence of second tasks, a user performance characteristic based on spatial and temporal properties of the one or more user inputs on touchscreen display; and
evaluating, for each respective second task of the sequence of second tasks, a cognitive ability of the user based on a comparison of the determined performance characteristic of the respective second task with the determined performance characteristic of a preceding second task or the determined performance characteristics of a plurality of preceding second tasks of the sequence of the second tasks.

13. The method according to any one of the preceding claims, further comprising detecting motion of the mobile device within the one or more first task(s) and associated with related to the one or more user inputs related to the one or more first objects and/or the one or more second objects on the touchscreen display, wherein determining the user performance characteristic is further based on the detected motion of the mobile device.

14. An apparatus comprising:
a touchscreen display; and
a processor configured to perform a method according to any one of claims 1 to 14.

15. Computer program product comprising instructions that, when executed by a processor of an apparatus according to claim 14, cause the processor to perform a method according to any one of claims 1 to 13.
